(19) Europäisches Patentamt
European Patent Office
Office européen des brevets

(11) EP 2 896 701 A1

(12) **EUROPEAN PATENT APPLICATION**
published in accordance with Art. 153(4) EPC

(43) Date of publication:
**22.07.2015 Bulletin 2015/30**

(21) Application number: **13837548.0**

(22) Date of filing: **05.09.2013**

(51) Int Cl.:
***C12P 7/62*** *(2006.01)* ***C12N 1/00*** *(2006.01)*

(86) International application number:
**PCT/JP2013/073982**

(87) International publication number:
**WO 2014/042076 (20.03.2014 Gazette 2014/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.09.2012 JP 2012203477**

(71) Applicants:
- **Tokyo Institute of Technology**
  **Tokyo 152-8550 (JP)**
- **Kaneka Corporation**
  **Kita-ku**
  **Osaka 530-8288 (JP)**

(72) Inventors:
- **TAGO, Masako**
  **Tokyo 152-8550 (JP)**
- **TSUGE, Takeharu**
  **Tokyo 152-8550 (JP)**
- **SATO, Shunsuke**
  **Takasago-shi**
  **Hyogo 676-8688 (JP)**

(74) Representative: **Hoffmann Eitle**
**Patent- und Rechtsanwälte PartmbB**
**Arabellastraße 30**
**81925 München (DE)**

# (54) METHOD FOR PRODUCING POLYHYDROXYALKANOATE USING MODIFIED FAT OR OIL COMPOSITION

(57) The present invention aims to provide a plant-derived carbon source modified for production of a high molecular weight polyhydroxyalkanoate having excellent processing properties, and a method for producing polyester using the carbon source. Specifically, the present invention aims to provide a technique for efficiently removing fatty acids such as butanoic acid, pentanoic acid, and hexanoic acid present in a fraction having a high free fatty acid content of a plant-derived carbon source; and a method for producing a polyhydroxyalkanoate by microorganisms. It was found that activated clay treatment of a fraction having a high free fatty acid content of a carbon source derived from a vegetable fat and/or oil can efficiently remove butanoic acid, pentanoic acid, and hexanoic acid; and that culturing microorganisms using a carbon source treated with activated clay can produce a higher molecular weight polyhydroxyalkanoate. Provided is a method for producing a polyhydroxyalkanoate, the method including culturing a microorganism with a plant-derived carbon source containing butanoic acid, pentanoic acid, and hexanoic acid in a total amount of 50 mM or less and having a free fatty acid content of 20% by weight or more.

EP 2 896 701 A1

## Description

TECHNICAL FIELD

The present invention relates to a technique of a method for producing a polyhydroxyalkanoate (hereinafter referred to as PHA).

BACKGROUND ART

In view of increased awareness of environmental problems, food problems, health and safety issues, and orientation toward non-synthetic and natural products, production of microorganisms and microbial production of substances (such as fermentative production and bioconversion) are increasingly gaining significance and importance.

The production of microorganisms and the microbial production of substances require a carbon source (carbon source for culture, fermentation, or the like) to be suitably assimilated by microorganisms. Examples of representative carbon sources include carbohydrates, fats and oils (such as vegetable fats and oils), and fatty acids (such as plant-derived fatty acids). In view of recent environmental problems and other issues, these carbon sources have been desired to be renewable (particularly, non-petroleum carbon sources) and, moreover, non-competitive with food (i.e., non-edible carbon sources).

From these viewpoints, fatty acids (for example, plant-derived fatty acids) are considered to be one suitable alternative for carbon sources. These fatty acids can be obtained from, for example, coconut and palm oils (including palm kernel oil). Representative fatty acids derived from these plants include long-chain fatty acids including long-chain saturated fatty acids such as C12 lauric acid, C14 myristic acid, and C16 palmitic acid, and long-chain unsaturated fatty acids such as C18 oleic acid.

Examples of polyester type organic molecular polymers produced by a wide variety of microorganisms using carbon sources such as fatty acids include a PHA, which is a biodegradable thermoplastic polymer and is also producible from renewable resources. Thus, attempts have been made to industrially produce a PHA as an environmentally friendly material or biocompatible material for various industrial applications. For example, studies have been made to produce a PHA using palm fatty acid distillate (PFAD) which is a fatty acid mixture derived from a palm oil refining process or palm kernel acid oil (PKAO) which is a fatty acid mixture derived from palm kernel oil (Non-Patent Literature 1).

The properties of PHA are dependent on the molecular weight. A PHA having as high a molecular weight as possible is preferred for fiber processing. Thus, a technique to control the molecular weight of PHA, particularly to increase the molecular weight of PHA, in a fermentative production process has been required.

Some of the substances that strongly affect the molecular weight of PHA produced by microorganisms have been reported. For example, it has been reported that when glucose and various organic compounds were added as carbon sources to Ralstonia eutropha (currently classified as Cupriavidus necator), a compound having a hydroxyl group, such as 1-propanol, 1-butanol, 1,4-butanediol, glycerol, and ethylene glycol exhibited an effect of reducing the molecular weight of PHA (Non-Patent Literature 2). Yet, it is unlikely that fatty acid mixtures which are by-products of refined fats and oils contain a substance having a hydroxyl group, and thus, there are still problems in the technique of producing a PHA by utilizing an industrially useful plant-derived fatty acid mixture while suppressing a reduction in the molecular weight.

One of fatty acid mixtures derived from vegetable oils is a composition derived from industrially important palm kernel oil (hereinafter, the composition is referred to as PKFAD, short for palm kernel fatty acid distillate (synonymous with PKAO)) (Non-Patent Literature 3). Yet, the usefulness of PKFAD in microbial production of a PHA is unknown.

CITATION LIST

NON PATENT LITERATURES

Non-Patent Literature 1: Appl Microbiol Biotechnol, 89: 1373-1386 (2011)
Non-Patent Literature 2: Biological Macromolecules, 25: 43-53 (1999)
Non-Patent Literature 3: Journal of Oleo Science 56 (1) : 13-17 (2007)

SUMMARY OF INVENTION

TECHNICAL PROBLEM

The present invention aims to provide a method for producing a high molecular weight PHA by microorganisms. Further, the present invention aims to provide a modified plant-derived carbon source, and a method for producing a PHA using the carbon source. Specifically, the present invention aims to provide a carbon source obtained by removing a substance that is present in a plant-derived carbon source and that causes a reduction in the molecular weight of PHA to be synthesized by microorganisms; and the present invention also aims to provide a method for producing a PHA using the carbon source.

SOLUTION TO PROBLEM

The present inventors conducted studies on PHA production using palm kernel fatty acid distillate (PKFAD) derived from palm kernel oil and having a high free fatty acid content. As a result, they found that the use of such PKFAD can produce a PHA having a low molecular weight, compared to the case where palm kernel oil (PKO) having a low free fatty acid content is used. Further, they found that use of a clay-treated plant-derived carbon source results in an increase in the molecular weight of PHA to be produced by microorganisms; and still further, they found that substances selectively removed by clay treatment are butanoic acid, pentanoic acid, and hexanoic acid, and that these acids actually cause a reduction in the molecular weight. The present invention was accomplished based on these findings.

The present invention relates to a method for producing a polyhydroxyalkanoate, the method including culturing a microorganism with a plant-derived carbon source containing butanoic acid, pentanoic acid, and hexanoic acid in a total amount of 50 mM or less and having a free fatty acid content of 20% by weight or more.

Preferably, the method further includes a step of preparing the plant-derived carbon source containing butanoic acid, pentanoic acid, and hexanoic acid in a total amount of 50 mM or less from a plant-derived carbon source containing butanoic acid, pentanoic acid, and hexanoic acid in a total amount of 50 mM or more and having a free fatty acid content of 20% by weight or more.

The plant-derived carbon source preferably contains 6 mM or less of butanoic acid.

The plant-derived carbon source preferably contains 35 mM or less of hexanoic acid.

The plant-derived carbon source is preferably treated with activated clay.

The plant-derived carbon source is preferably palm kernel oil.

The microorganism is preferably a bacterium of the genus Cupriavidus.

The bacterium of the genus Cupriavidus is preferably Cupriavidus necator or its recombinant.

The polyhydroxyalkanoate is preferably polyhydroxybutyrate-hexanoate.

The present invention also relates to a method for producing a carbon source for culturing a microorganism, the method including contacting a plant-derived carbon source with activated clay.

ADVANTAGEOUS EFFECTS OF INVENTION

The present invention enables production of a high molecular weight PHA by utilizing an industrially significant carbon source produced in a production process of vegetable fats and/or oils.

DESCRIPTION OF EMBODIMENTS

The present invention is described in detail below.

The type of PHA of the present invention is not particularly limited as long as it is produced by microorganisms. Yet, preferably, it is a PHA obtained by polymerization of a constituent component selected from C4-C16 3-hydroxyalkanoic acids, or a copolymerized PHA obtained by copolymerization of constituent components selected from C4-C16 3-hydroxyalkanoic acids.

Examples include polyhydroxybutyrate P(3HB) obtained by polymerization of a C4 3-hydroxyalkanoic acid, polyhydroxybutyrate-hexanoate P(3HB-co-3HH) obtained by copolymerization of C4 and C6 3-hydroxyalkanoic acids, polyhydroxybutyrate-valerate P(3HB-co-3HV) obtained by copolymerization of C4 and C5 3-hydroxyalkanoic acids, and polyhydroxyalkanoate (PHA) obtained by polymerization or copolymerization of C4-C14 3-hydroxyalkanoic acids. The type of PHA to be produced can be suitably selected when introducing a known gene involved in PHA synthesis into, for example, C. necator used as a host microorganism.

In the present invention, a plant-derived carbon source is used for culturing microorganisms. The plant-derived carbon source may contain a fatty acid mixture. The fatty acid mixture refers to a mixture of hydrophobic organic substances such as triacylglycerol, diacylglycerol, monoacylglycerol, and a free fatty acid.

The plant-derived carbon source may be a carbon source derived from any plant as long as the carbon source can be assimilated by PHA-producing microorganisms. Although the plant is not limited, a preferred plant-derived carbon source contains a large amount of free fatty acids, which is removed by distillation in a production process of fats and/or oils such as palm oil, palm kernel oil, corn oil, coconut oil, olive oil, soybean oil, rapeseed oil, and jatropha oil. Palm kernel oil, coconut oil, and other like vegetable oils are more preferred because they contain shorter chain fatty acids as constituent fatty acids.

The plant-derived carbon source contains butanoic acid, pentanoic acid, and hexanoic acid in a total amount of 50 mM or less, preferably 42 mM or less, more preferably 40 mM or less, still more preferably 20 mM or less. The amount of butanoic acid and the amount of pentanoic acid are preferably 6 mM or less, more preferably 4 mM or less, still more preferably 2 mM or less. The amount of hexanoic acid is preferably 35 mM or less, more preferably 30 mM or less, still more preferably 25 mM or less.

PFAD and PKFAD, which are expected as non-edible useful carbon sources, may sometimes contain butanoic acid, pentanoic acid, and hexanoic acid, which are organic acids, in a total amount of more than 50 mM. If the total amount of butanoic acid, pentanoic acid, and hexanoic acid in the plant-derived carbon source is large, the carbon source can be modified to prepare a plant-derived carbon source containing butanoic acid, pentanoic acid, and hexanoic acid in a total amount of 50 mM or less. Examples of preparation methods include a method for removing the organic acids from the carbon source. Any removal method may be used as long as the organic acids can be removed. Examples include activated clay treatment, activated carbon treatment, extraction method using a water-soluble component such as water, and a combination of these methods. Alternatively, the carbon source containing the organic acids in a total amount of more than 50 mM may be mixed (diluted) with another carbon source such as palm Kernel oil (PKO) or palm olein oil (POO) to thereby prepare a plant-derived carbon source containing the organic acids in a total amount of 50 mM or less while maintaining the free fatty acid content in the entire carbon source at 20% by weight or more. Among these preparation methods, the method for removing the organic acids is preferred, and the activated clay treatment in which the plant-derived carbon source is contacted with activated clay is particularly preferred.

The long chain free fatty acid content in the plant-derived carbon source is 20% by weight or more, preferably 50% by weight or more, more preferably 80% by weight or more, in view of industrial applications of a non-edible component having a high free fatty acid content. The term "free fatty acids" as used herein refers to fatty acids that are not bound to other compounds. Examples of long chain free fatty acids include caprylic acid, capric acid, lauric acid, myristic acid, palmitic acid, stearic acid, oleic acid, linoleic acid, linolenic acid, arachidic acid, and palmitoleic acid.

Any microorganism may be used for PHA production as long as it is a microorganism capable of synthesizing a PHA or its recombinant. Examples include bacteria of the genera Ralstonia, Cupriavidus, Wautersia, Aeromonas, Escherichia, Alcaligenes, Pseudomonas, and the like. Among these, bacteria of the genus Cupriavidus are preferred. Among bacteria of the genus Cupriavidus, Cupriavidus necator is more preferred. A mutant strain obtainable by artificially mutating the microorganism, and a recombinant obtained by mutating the microorganism through genetic engineering can also be used.

Examples of the gene involved in PHA synthesis to be introduced into the host microorganism include genes for thiolase and reductase constituting a 3HB supplying system; PHB synthesizing enzyme (i.e., PHB synthase); PHA synthesizing enzyme (i.e., PHA synthase); and acyl-CoA transferase, enoyl-CoA hydratase, and acyl-CoA dehydrogenase, which are enzymes in the β oxidation pathway. The thiolase may includeβ -ketothiolase; the reductase may include acetoacetyl-CoA reductase; the PHA synthase may include Aeromonas caviae-derived PHA synthase mutant gene (i.e., N149S/D171G mutant); and the acyl-CoA transferase may include 3-hydroxyacyl-ACP-CoA transferase. These genes are preferably derived from PHA-producing bacteria, such as Aeromonas caviae, Aeromonas hydrophila, and the genus Chromobacterium. A mutation may be introduced into these genes.

In the case of using a plasmid for introducing a gene into the host microorganism, the plasmid is preferably one capable of stable replication and maintenance in the host microorganism even when no selective pressure is present. Examples of such plasmids include pCUP2 disclosed in PCT publication No. WO 2007/049716. The pCUP2 has a region necessary for replication and stable maintenance of a plasmid derived from megaplasmid pMOL28 of Cupriavidus metallidurans CH34, which is closely related to C. necator. Meanwhile, a vector may contain a drug-resistance gene for stable replication and maintenance in the host microorganism.

A method for introducing a gene into the host microorganism to prepare a transformant is not particularly limited, and any known method can be used. For example, methods such as electroporation method, calcium method, and conjugal transfer method can be used.

After microorganisms are grown in a preculture medium, the microorganisms are cultured in a PHA-producing medium, thus allowing accumulation of PHAs in the microorganisms. The preculture medium is not particularly limited as long as it is a medium in which microorganisms can be grown.

The PHA-producing medium contains a plant-derived carbon source, and may also contain a nitrogen source, an inorganic salt, and the like. Examples of the nitrogen source include ammonia and ammonium salts such as ammonium chloride, ammonium sulfate, and ammonium phosphate. Examples of inorganic salts include potassium dihydrogen

phosphate, disodium hydrogen phosphate, magnesium phosphate, magnesium sulfate, and sodium chloride. The medium may contain an antibiotic (for example, kanamycin) that corresponds to the drug-resistance gene contained in the gene expression plasmid.

The culture may be carried out at any temperature as long as the microorganisms can grow. A preferred temperature range is from 20°C to 40°C. The culture period is not particularly limited, and may be about 1 day to 10 days.

PHAs can be recovered from the microorganisms by the following methods, for example. After culturing, the cells are separated from the culture fluid using a centrifuge or the like, and the cells are washed with distilled water and methanol or the like, and then dried; and the PHAs are extracted from the dried cells using an organic solvent such as chloroform. Cell components are removed by filtration or the like from the solution containing the PHAs, and a poor solvent such as methanol or hexane is added to the filtrate to precipitate the PHAs. Alternately, the supernatant is removed by filtration or centrifugation, and after drying, the PHAs are recovered.

The yield of cells can be measured by a known method such as optical density method or dry cell weight measurement method, and the yield of the substance produced by the microorganisms can be measured by a known method such as GC or HPLC. The PHA content accumulated in the cells can be measured after extracting the PHAs from the cultured cells using an organic solvent such as chloroform and drying the extract, according to the method of Kato et al. (Appl. MicroBiol. Biotechnol., vol. 45, p. 363 (1996); Bull. Chem. Soc., vol. 69, p. 515 (1996)).

EXAMPLES

The present invention is described in detail below with reference to examples, but the present invention is not limited to these examples. The general gene manipulation can be carried out as described in Molecular Cloning (Cold Spring Harbor Laboratory Press (1989)). Enzymes, cloning hosts and other materials to be used in gene manipulation can be purchased from commercially available suppliers and can be used according to the instructions given by the suppliers. The enzymes are not particularly limited as long as they can be used in gene manipulation.

(Example 1)

(1) Modification of PKFAD by clay treatment

A carbon source PKFAD was purchased from MALAYSIAN BIOTECHNOLOGY CORPORATION SDN BDH. One kilogram of PKFAD was weighed out and subjected to a high-temperature reduced-pressure treatment at 90°C for 10 minutes in an evaporator (available from EYELA, N-1200 model). Subsequently, 10 wt% of activated clay (available from Mizusawa Industrial Chemicals, Ltd.) was added to PKFAD, and a high-temperature reduced-pressure treatment was carried out again at 90°C for 30 minutes in an evaporator of the same type. Thereby, fatty acid components such as butanoic acid, pentanoic acid, and hexanoic acid in PKFAD were adsorbed onto the clay. Subsequently, the clay was removed by filtration, whereby a modified vegetable fat and/or oil (hereinafter referred to as "modified PKFAD") was prepared.

(2) Analysis of fatty acids in PKFAD and modified PKFAD

Quantitative analysis of fatty acids in the PKFAD, modified PKFAD, and triolein (available from Wako Pure Chemical Industries, Ltd. ; used as a standard carbon source in Reference Example 1) was carried out. The fatty acid content was analyzed by an effective carbon number method using a flame ionization detector (FID).

(2-1) Sample preparation

The PKFAD and modified PKFAD were filtered through a polyvinylidene fluoride (PVDF) filter ($\phi$ = 0.45 μm, available from Advantec). The resulting products (500 μL each) were individually mixed with 500 μL of hexane (internal standard) in a 1.5-mL vial tube, which was sealed with an 8-mm polytetrafluoroethylene (PTFE)/silicone septum.

(2-2) Measurement conditions

GC was carried out under the following conditions. Device
GC device: gas chromatograph GC-14B (available from Shimadzu Corporation)
Capillary column: 100% methylpolysiloxane non-polar column 1010-11143 (cross-linked, internal diameter of 0.25 mm x 30 mm, film thickness of 0.4 μm) (available from GL Sciences Inc.) Detector: flame ionization detector (FID)

Autoinjector: AOC-20i Autoinjector 221-44527-30 (available from Shimadzu Corporation)
Autosampler: AOC-20s autosampler 221-44880-30 (available from Shimadzu Corporation)
Analyzer: Chromatopac C-R7A plus (available from Shimadzu Corporation)

Table 1 shows gas pressures.

[Table 1]
Gas pressure during measurement

| Gas | Pressure [kPa] |
|---|---|
| $N_2$ | 80 |
| He | 120 |
| $H_2$ | 50 |
| Air | 50 |

Table 2 shows temperature setting.

[Table 2]
Temperature setting during measurement

| Setting item | Setting value |
|---|---|
| Detector temperature | 280°C |
| Injector temperature | 280°C |
| Column oven temperature | 100°C |
| Initial temperature holding time | 2 min |
| Column temperature increase rate | 8°C/min |
| Column final temperature | 280°C |
| Final temperature holding time | 5 min |

(2-3) GC data analysis

The effective carbon number method using an FID is a method for calculating the molar concentration of a substance. This method is based on the concept that the relative molar response of a hydrocarbon is proportionate to the number of carbon atoms and that in the case of hydrocarbon derivatives in which hydrogen atoms of a hydrocarbon are replaced with other atoms or functional groups, the relative molar response is proportionate to the sum of effective carbon atoms determined by adding or subtracting predetermined correction values to or from the number of carbon atoms to which these other atoms or functional groups are bonded. Table 3 shows the effective carbon number for each carbon atom in each type and the correction value of each atom bonded to one carbon atom according to Sternberg et al. (Sternberg, J. C., Gallaway, W. E., and Jones, D. T. L.: Gas Chromatography. Academic Press, New York: 207 (1962)).

Effective carbon number and correction value

[Table 3]

| Atom | Type | Effective carbon number | Atom | Type | Effective carbon number |
|---|---|---|---|---|---|
| C | Saturated aliphatic | 1.0 | O | Tertiary alcohol | -0.25 |
| C | Aromatic | 1.0 | O | Ester | -0.25 |
| C | Olefinic | 0.95 | Cl | One atom bonded to Cl atom of saturated aliphatic | -0.0 |
| C | Acetylenic | 1.30 | Cl | Two or more atoms bonded to Cl atom of saturated aliphatic | -0.12 (each) |

(continued)

| Atom | Type | Effective carbon number | Atom | Type | Effective carbon number |
|---|---|---|---|---|---|
| C | Carbonyl | 0.0 | Cl | Bonded to olefinic C | +0.05 |
| C | Nitrile | 0.3 | | | |
| O | Ether | -1.0 | N | Primary amine | -0.6 |
| O | Primary alcohol | -0.6 | N | Secondary amine | -0.75 |
| O | Secondary alcohol | -0.75 | N | Tertiary amine | -0.25 |

For example, for calculation of the molar concentration of hexanoic acid, the effective carbon number of hexanoic acid is determined by the following formula: (saturated aliphatic) + (carbonyl) - (primary alcohol), i.e., 1.0x5+0.0-0.6=4.4.

Table 4 shows analysis results.

[Table 4]

Results of quantitative analysis of fatty acids in PKFAD and modified PKFAD

| | PKFAD (mM) | Modified PKFAD (mM) | Triolein |
|---|---|---|---|
| Butanoic acid | 6.2 | 3.4 | 0 |
| Pentanoic acid | 5.7 | 5.4 | 0 |
| Hexanoic acid | 40 | 33 | 0 |

(Reference Example 1) Culture with addition of butanoic acid, pentanoic acid, and hexanoic acid

The results of Example 1 clearly show that the concentrations of butanoic acid, pentanoic acid, and hexanoic acid were reduced in the modified PKFAD. Thus, the effect of the addition of the fatty acids to reduce the molecular weight of PHA was examined.

(1) Strain

The following strains were used.

· Escherichia coli S17-1 (see Simon, R., Priefer, U., and Puhler, A.: A broad host range mobilization system for in vivo genetic engineering transposon mutagenesis in gram negative bacteria. Biotechnology, 1: 784-791 (1983))
· d1 (see JP-A 2007-259708)

(2) Plasmid

The following plasmid was used.

- pBBREE 32d13dPB NSDG

(See Watanabe, Y., Ichinomiya, Y., Shimada, D., Saika. A., Abe, H., Taguchi, S., and Tsuge, T.: Development and validation of an HPLC-based screening method to acquire polyhydroxyalkanoate synthase mutants with altered substrate specificity. J Biosci Bioeng. 113:286-292(2011))

(3) Medium

All the media were sterilized by autoclave at 121°C and 2 atmospheres for 20 minutes. A reagent to be added to each medium composition after autoclave was sterilized by filtration through a 0.20 μm filter. In the case of preparing an agar plate medium, agar was added at 1.5% (w/v). In the case of adding an antibiotic, kanamycin was added to the autoclaved media to a final concentration of 50 μg/mL.

(3-1) Luria-Bertani (LB) medium

The strain E. coli S17-1 used for conjugal transfer of genes was cultured in LB medium. Table 5 shows the composition of LB medium. An antibiotic was added after autoclave.

[Table 5]

LB medium

| Component | Concentration ($L^{-1}$) |
|---|---|
| Bacto trypton | 10 g |
| Yeast extract | 5 g |
| NaCl | 10 g |

(3-2) Nutrient-Rich (NR) medium

The strain d1 used for conjugal transfer and preculture was cultured in NR medium. Table 6 shows the composition of NR medium. An antibiotic was added after autoclave.

[Table 6]

NR medium

| Component | Concentration ($L^{-1}$) |
|---|---|
| Bacto trypton | 10 g |
| Yeast extract | 2 g |
| Meat extract | 10 g |

(3-3) Simmons citrate medium

Simmons citrate medium was used to screen the strain d1 for conjugal transfer. Table 7 shows the composition of Simmons citrate medium. An antibiotic was added after autoclave.

[Table 7]

Simmons citrate medium

| Component | Concentration ($L^{-1}$) |
|---|---|
| Trisodium citrate dehydrate | 2 g |
| NaCl | 5 g |
| $KH_2PO_4$ | 1 g |
| $NH_4H_2PO_4$ | 1 g |
| 0.2 g/mL $MgSO_4$ soln. | |

(3-4) Mineral salt (MS) medium

The strain d1 for PHA biosynthesis was cultured in MS medium. Tables 8 and 9 each show the composition of MS medium. In the case of culturing using triolein as a carbon source, 20 g/L of triolein was added before autoclave. Trace metals, magnesium sulfate, and a chain transfer agent were added after autoclave. The fatty acids were added to a final concentration of 1 mM in the entire medium and to a concentration of 50 mM in triolein.

[Table 8]

MS medium

| Component | Concentration ($L^{-1}$) |
|---|---|
| $Na_2HPO_4 \cdot 12H_20$ | 9 g |
| $KH_2PO_4$ | 1.5 g |
| $NH_4Cl$ | 0.5 g |

(continued)

| MS medium | |
|---|---|
| Component | Concentration ($L^{-1}$) |
| NaCl | 0.5 g |
| 0.2 g/mL $MgSO_4$ soln. | 1 mL |
| Trace element soln. | 0.1 mL |
| | pH = 7.0 |

[Table 9]

Trace element

| Component | Concentration ($L^{-1}$) |
|---|---|
| $CoCl_2 \cdot 6H_2O$ | 0.218 g |
| $FeCl_3$ | 9.7 g |
| $CaCl_2$ | 7.8 g |
| $NiCl_3 \cdot 6H_2O$ | 0.118 g |
| $CrCl_3 \cdot 6H_2O$ | 0.105 g |
| $CuSO_4 \cdot 5H_2O$ | 0.156 g |

(3-5) SOC medium

SOC medium was used for transformation in a calcium chloride method. Table 10 shows the composition of SOC medium.

[Table 10]

SOC element

| Component | Concentration ($L^{-1}$) |
|---|---|
| Bacto trypton | 5 g |
| Yeast extract | 0.5 g |
| 250 mM KCl | 10 mL |
| 2 M $MgCl_2$ soln. | 5 mL |
| 1 M glucose soln. | 20 L |

(4) Transformation

(4-1) Calcium chloride method

The target gene was subcloned by the calcium chloride method.

(i) Competent cells of E. coli S17-1 were thawed on ice, and a plasmid DNA (pBBREE32d13dPB NSDG) solution (2 μL) was added gently.
(ii) After cooling on ice for 30 to 60 minutes, the cells were heat shocked in a constant temperature bath at 42°C for 30 to 90 seconds.
(iii) After heat shock, the cells were immediately transferred onto ice for cooling for 2 to 3 minutes.
(iv) SOC medium (900 μL) was added, and shake culture was carried out at 37°C for 30 to 60 minutes.
(v) The cells were spread on LB agar medium containing kanamycin, followed by culture at 37°C overnight.

(4-2) Conjugal transfer method

Conjugal transfer was carried out in order to introduce the gene into d1.

(i) The strain E.coli S17-1 into which the target plasmid was introduced by transformation was inoculated into LB

medium (1. 7 mL) containing kanamycin, followed by culture at 37°C for 16 hours.

(ii) The host (d1) into which the gene was introduced was also inoculated into NR medium (1. 7 mL), followed by culture at 30°C for 16 hours.

(iii) After culturing, the entire amount of each culture fluid was transferred into an autoclaved Eppendorf tube, and the fluid was centrifuged at 10,000 rpm for 1 minute.

(iv) LB medium (500 μL) was further added to the medium of E. coli S17-1 containing kanamycin, and centrifugation was carried out to completely remove the antibiotic.

(v) The supernatant was discarded leaving about 50 μL, and E. coli S17-1 and R. eutropha d1 were mixed. The mixture was then added dropwise to LB agar medium and dried, followed by culture at 30°C for 5 hours.

(vi) After culturing, the strains were streaked on Simmons citrate agar medium containing kanamycin, followed by culture at 30°C for 2 days.

(vii) The obtained single colony was streaked again in Simmons citrate medium containing kanamycin, followed by culture at 30°C for 2 days.

(viii) The formed single colony was streaked in NR agar medium containing kanamycin, followed by culture at 30°C for 2 days.

(ix) The formed single colony was inoculated into NR medium (1.7 mL) containing kanamycin, followed by culture at 30°C for 16 hours. Subsequently, the culture fluid (100 μL) was mixed with a 30% autoclaved glycerol solution (100 μL), and the mixture was stored at -80°C.

(5) Shake culture and harvest

(5-1) Culture method

NR medium and MS medium were used to culture d1/pBBREE32d13dPB NSDG. The glycerol stock solution prepared above was streaked in NR agar medium containing kanamycin, followed by culture at 30°C for 2 days. The formed single colony was inoculated into NR liquid medium (1.7 mL) containing kanamycin, followed by shake culture at 150 $min^{-1}$ at 30°C for 16 hours (preculture). MS liquid medium (50 mL) containing triolein (20 g/L) as a single carbon source was added to a 500-mL shake flask, and the preculture fluid (500 μL) was inoculated into the medium, followed by shake culture at 130 $min^{-1}$ at 30°C for 72 hours (main culture). The antibiotic added was kanamycin (50 mg/L).

(5-2) Harvest method

The main culture fluid was transferred to a 250-mL centrifuge tube. A shake flask was rinsed once with an adequate amount of hexane and three times with deionized water. These wash liquids were also added to the centrifuge tube, followed by vigorous stirring and then centrifugation at 6000 rpm at 4°C for 10 minutes to remove the supernatant. The cell pellets were suspended in an adequate amount of deionized water, followed by vigorous stirring and then centrifugation at 6000 rpm for 10 minutes to remove the supernatant. Pellets were suspended in an adequate amount of deionized water (less than 5 mL), and the suspension was transferred to a harvest tube (5 mL) of a known tare weight. The tube was then sealed with parafilm and frozen at -20°C. Several vent holes were made in the parafilm sealing the opening of the tube, and the moisture was completely removed by a vacuum dryer (approximately 3 days). The parafilm was removed, and the tube was weighed, whereby the dry cell weight was determined.

(6) Analysis of polymer

(6-1) PHA content and analysis of monomer composition ratio

The PHA content synthesized by the cultured cells and the monomer composition ratio were determined by gas chromatography (GC) using an internal standard method (see Sternberg, J. C., Gallaway, W. E., and Jones, D. T. L.: Gas Chromatography. Academic Press, New York: 207 (1962)). With this method, methyl ester as a PHA degradation product in dried cells produced by methanolysis reaction is analyzed.

Degradation of PHA by methanolysis reaction

[Chem. 1]

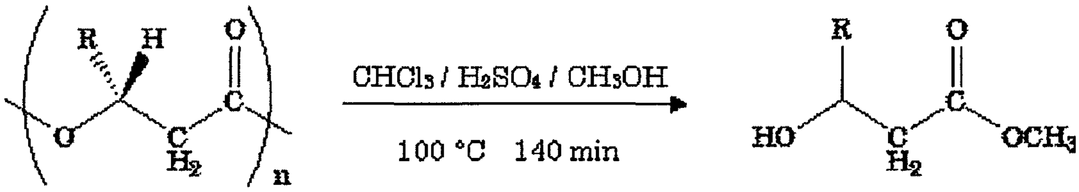


(6-2) Sample preparation for GC

About 10 mg of the dried cells was weighed into a test tube having a threaded lid, and a 15% sulfuric acid-methanol solution (2mL) and chloroform (2 mL) were added thereto. Then, the mixture was heated at 100°C for 140 minutes with vigorously stirring several times while heating. After the mixture was cooled to room temperature, ultra pure water (1 mL) was added to the mixture with vigorous mixing. Then, the mixture was left overnight. The lower layer (chloroform layer) of the sample solution, which has separated into two layers, was extracted with a Pasteur pipette, and the extract was filtered through a polyvinylidene fluoride (PVDF) filter (ϕ = 0.45 μm, available from Advantec). The filtrate (500 μL) and a 0.1 vol% methyl octanoate solution in chloroform (500 μL) were added to a 1. 5-mL vial tube, and the vial tube was sealed with an 8-mm polytetrafluoroethylene (PTFE)/silicone septum.

(6-3) GC

GC was carried out under the following conditions.

· Device

GC device: gas chromatograph GC-14B (available from Shimadzu Corporation)
Capillary column: 100% methylpolysiloxane non-polar column 1010-11143 (cross-linked, internal diameter of 0.25 mm × 30 m, film thickness of 0.4 μm) (available from GL Sciences Inc.)) Detector: flame ionization detector (FID)
Autoinjector: AOC-20i autoinjector 221-44527-30 (available from Shimadzu Corporation)
Autosampler: AOC-20s autosampler 221-44880-30 (available from Shimadzu Corporation)
Analyzer: Chromatopac C-R7A plus (available from Shimadzu Corporation)

Table 11 shows gas pressures.

[Table 11]
Gas pressure during measurement

| Gas | Pressure [kPa] |
|---|---|
| $N_2$ | 80 |
| He | 120 |
| $H_2$ | 50 |
| Air | 50 |

Table 12 shows set temperatures.

[Table 12]
Temperature setting during measurement

| Setting item | Setting value |
|---|---|
| Detector temperature | 280°C |
| Injector temperature | 280°C |
| Column oven temperature | 100°C |
| Initial temperature holding time | 0 min |

(continued)

| Temperature setting during measurement | |
|---|---|
| Setting item | Setting value |
| Column temperature increase rate | 8°C/min |
| Column final temperature | 280°C |
| Final temperature holding time | 5 min |

(6-4) GC data analysis

The PHA content in the cells and the monomer composition ratio were determined from the integrated peak values corresponding to each retention time detected by the FID. The results from GC were analyzed by the following procedure.

In the case where methyl esters a, b, and c corresponding to each monomer are detected by the results of GC, provided that the respective integrated values are given as A, B, and C and that the corresponding correction factors (reciprocal of the relative response) are given as X, Y, and Z, then the mole fraction of the methyl ester a can be determined from the following formula.

[Math 1]

$$a(mol\%) = \frac{(A \times X)}{(A \times X + B \times Y + C \times Z)} \times 100$$

Table 13 shows the correction factors standardized by the value of 3HB.

[Table 13]

| Correction factor of methyl 3-hydroxyalkanoate | |
|---|---|
| | Correction factor |
| Methyl 3-hydroxybutyrate | 1 |
| Methyl 3-hydroxyhexanoate | 0.51 |

The PHA content P (wt%) in the cells can be calculated by the following formula, where S is the integrated peak value of methyl octanoate as an internal standard substance and m (mg) is the weight of the dried cells used in sample.

[Math 2]

$$P(wt\%) = K \times \frac{(A \times X + B \times Y + C \times Z)}{(m \times S)} \times 100$$

Herein, K is a constant determined from a calibration curve prepared using P(3HB), and the value of K was calculated to be 6.2 by the following formula.

[Math 3]

$$K = [\text{weight of } P(3HB)] \times \frac{S}{(\text{Integrated peak value of 3HB})}$$

(7) GPC measurement

(7-1) Extraction of polymer

The dried cells were transferred to a 50-mL threaded sample tube. Chloroform (50 mL) was added to the tube, and the mixture was stirred at room temperature for 3 days. Subsequently, the solution was filtered (No. 1 filter paper available from ADVANTEC) to remove the cells. Then, the solvent was removed by a rotary evaporator, whereby a crude PHA was obtained.

(7-2) Purification of polymer

The crude PHA was completely dissolved in an adequate amount of chloroform, and the solution was added dropwise to methanol in about 10 times its volume of PHA. The mixture was left to stand, and then the precipitate was separated by filtration (No. 5B filter paper available from ADVANTEC). Subsequently, the solvent was completely removed by a vacuum dryer, whereby a purified PHA was obtained.

(7-3) GPC sample preparation method

The PHA (approximately 1 mg) was placed in a 5-mL vial tube, and chloroform (1 mL) was added to the vial tube. The mixture was left overnight to dissolve the PHA. The PHA solution was filtered through PVDF filter ($\phi$ = 0.45 $\mu$m, available from Advantec), and the entire amount of the filtrate was placed in a 1.5-mL vial tube, which was then sealed with an 8-mm PTFE/silicone septum.

(7-4) GPC measurement conditions

• Device

System controller: SCL-10A VP (available from Shimadzu Corporation)
Autoinjector: SIL-10A VP (available from Shimadzu Corporation)
Pump unit: LC-10AD VP (available from Shimadzu Corporation)
Column oven: CTO-10A (available from Shimadzu Corporation)
Separation column: K-806M, K-802 (available from Shodex)
Detector: RID-10A (available from Shimadzu Corporation)

• Measurement conditions

Table 14 shows measurement conditions.

[Table 14]

GPC conditions

| Conditioning factors | Setting value |
|---|---|
| Flow rate | 0.8 mL/min |
| Column oven temperature | 40°C |
| Sample injection amount | 60 $\mu$L |

(7-5) GPC data analysis

For analysis of the molecular weight and molecular weight distribution of the measurement sample, analysis software GPC for CLASS VP (available from Shimadzu Corporation) was used to calculate the weight average molecular weight (Mw) as a value relative to standard polystyrene as a standard substance.

Table 15 shows the results. The results clearly show that addition of butanoic acid, pentanoic acid, and hexanoic acid reduces the molecular weight (Mw) of PHA to be produced by microorganisms.

Results of evaluation of the effect on the molecular weight of PHA by addition of butanoic acid, pentanoic acid, and hexanoic acid

[Table 15]

| Carbon source | DCW (g/L) | content (%) | composition (mol%) | | Mw(x10^4) |
|---|---|---|---|---|---|
| | | | 3HB | 3HH | |
| Triolein : 20g/L | 3.6±0.4 | 83.7±6.1 | 97.0±0.1 | 3.0±0.1 | 172 |
| Triolein :20g/L+Butanoic acid 1 mM | 3.5±0.1 | 79.4±3.8 | 96.9±0.1 | 3.1±0.1 | 100 |
| Triolein :20g/L+ Pentanoic acid 1 mM | 3.7±0.2 | 80.1±9.1 | 96.6±0.0 | 3.4±0.0 | 100 |
| Triolein :20g/L+ Hexanoic acid 1 mM | 3.7±0.2 | 75.1±2.3 | 96.6±0.1 | 3.4±0.1 | 118 |

(Example 2) PHA production using modified PKFAD

The modified PKFAD prepared in Example 1 was used to produce a PHA by high-density culture. The strain Pac-bktB/AS+pCUP2-631 (see PCT publication No. WO 2009/145164) was used for production.

The composition of a seed culture medium was as follows: 1 w/v% Meat-extract, 1 w/v% Bacto-tryptone, 0.2 w/v% yeast extract, 0.9 w/v% $Na_2HPO_4 \cdot 12H_2O$, 0.15 w/v% $KH_2PO_4$, (pH 6.8).

The composition of the preculture medium was as follows: 1.1 w/v% $Na_2HPO_4 \cdot 12H_2O$ 0.19 w/v% $KH_2PO_4$, 1.29 w/v% $(NH_4)_2SO_4$, 0.1 w/v% $MgSO_4 \cdot 7H_2O$, 2.5 w/v% palm olein oil, 0.5 v/v% trace metal salt solution (0.1N hydrochloric acid containing 1.6 w/v% $FeCl_3 \cdot 6H_2O$, 1 w/v% $CaCl_2 \cdot 2H_2O$, 0.02 w/v% $CoCl_2 \cdot 6H_2O$, 0.016 w/v% $CuSO_4 \cdot 5H_2O$, and 0.012 w/v% $NiCl_2 \cdot {}_6H_2O$ dissolved therein). As a carbon source, palm olein oil was collectively added at a concentration of 10 g/L.

The composition of the PHA-producing medium was as follows: 0.385 w/v% $Na_2HPO_4 \cdot 12H_2O$, 0.067 w/v% $KH_2PO_4$, 0.291 w/v% $(NH_4)_2SO_4$, 0.1 w/v% $MgSO_4 \cdot 7H_2O$, 0.5 v/v% trace metal salt solution (0.1N hydrochloric acid containing 1. 6 w/v% $FeCl_3 \cdot 6H_2O$, 1 w/v% $CaCl_2 \cdot 2H_2O$, 0.02 w/v% $CoCl_2 \cdot 6H_2O$, 0.016 w/v% $CuSO_4.5H_2O$, and 0.012 w/v% $NiCl_2 \cdot {}_6H_2O$ dissolved therein).

First, a glycerol stock (50 μl) of Pac-bktB/AS+pCUP2-631 was inoculated into the seed culture medium (10 ml) and cultured for 24 hours for seed culture. Next, the seed culture fluid was inoculated at 1.0 v/v% into a 3-L jar fermenter (MDL-300 model available from B. E. MARUBISHI Co., Ltd.) containing 1. 8 L of preculture medium. Preculture was carried out by 24-culture under the following operating conditions: culture temperature of 28°C, stirring rate of 500 rpm, aeration rate of 1.8 L/min, and pH controlled in the range of 6.7 to 6. 8. The pH was controlled using a 14% aqueous ammonium hydroxide solution.

Next, the preculture fluid was inoculated at 5. 0 v/v% into a 5-L jar fermenter (MDS-U50 model available from B. E. MARUBISHI Co., Ltd.) containing 2.5 L of PHA-producing medium. The operating conditions were as follows: culture temperature of 28°C, stirring rate of 400 rpm, aeration rate of 2.1 L/min, and pH controlled in the ranged of 6.7 to 6.8. The pH was controlled using a 14% aqueous ammonium hydroxide solution. Modified PKFAD as a carbon source was added intermittently. Culture was carried out for 60 hours. After culturing, the cells were recovered by centrifugation, washed with methanol, and lyophilized. Then, the dry cell weight was measured.

Chloroform (100 ml) was added to the obtained dried cells (1 g), and the mixture was stirred at room temperature for 24 hours to extract PHA in the cells. The cell residue was separated by filtration, and the filtrate was concentrated in an evaporator to a total volume of 30 ml, followed by gradual addition of 90 ml of hexane. Then, the mixture was left for 1 hour with gentle stirring. Precipitated PHA was separated by filtration and vacuum dried at 50°C for 3 hours. The weight of dried PHA was measured, and the polymer content in the cells was calculated.

In addition, the molecular weight of the obtained PHA and the monomer composition ratio were measured by the same method as in Reference Example 1. Table 16 shows the results.

(Comparative Example 1) PHA production using unmodified PKFAD

PHA was produced and analyzed by the same method as in Example 2, except that unmodified PKFAD was used. Table 16 shows the results.

Results of high-density culture using modified PKFAD and unmodified PKFAD

[Table 16]

| Carbon source | DCW(g/L) | PHA(g/L) | PHA content (%) | PHA composition(mol%) | | Mw(x$10^4$) |
|---|---|---|---|---|---|---|
| | | | | 3HB | 3HH | |
| Example 2 Modified PKFAD | 132 | 101 | 77 | 88.4 | 11.6 | 105.5 |
| Comparative Example 1 PKFAD | 138 | 105 | 76 | 87.9 | 12.1 | 60.3 |

The results clearly show that the use of modified PKFAD containing small amounts of butanoic acid, pentanoic acid, and hexanoic acid can increase the molecular weight (Mw) of PHA to be produced by microorganisms.

(Measurement Example 1) Measurement of free fatty acid content in PFAD or PKFAD

The free fatty acid content in PFAD, PKFAD, and edible palm oils (palm kernel oil (PKO) and palm olein oil (PPO)) were measured in accordance with the Standard Methods for the Analysis of Fats, Oils and Related Materials "2.4.6.1-1996, Triacylglycerol composition (gas chromatography)". Table 17 shows the results.

The free fatty acid content in PKFAD, PFAD, and edible fats and/or oils (PKO and POO)

[Table 17]

| Sample | PFAD | PKFAD | PKO | POO |
|---|---|---|---|---|
| Free fatty acid (FFA) content (wt%) | 96.2 | 77.6 | 0.3 | 0.23 |
| Triglyceride (TG) content (wt%) | 0.90 | 0.77 | 99.4 | 94 |
| Diglyceride (DG) content (wt%) | 1.45 | 14.7 | 0 | 0.13 |
| Monoglyceride MG (wt%) | 0.62 | 4.4 | 0 | 5.77 |

PFAD and PKFAD produced in a refining process of fats and oils have a high free fatty acid content. According to the method of the present invention, high molecular weight PHA can be efficiently produced using a non-edible carbon source derived from a plant having a high free fatty acid content.

## Claims

**1.** A method for producing a polyhydroxyalkanoate, the method comprising:

culturing a microorganism with a plant-derived carbon source containing butanoic acid, pentanoic acid, and hexanoic acid in a total amount of 50 mM or less and having a free fatty acid content of 20% by weight or more.

**2.** The production method according to claim 1, further comprising a step of preparing the plant-derived carbon source containing butanoic acid, pentanoic acid, and hexanoic acid in a total amount of 50 mM or less from a plant-derived carbon source containing butanoic acid, pentanoic acid, and hexanoic acid in a total amount of more than 50 mM and having a free fatty acid content of 20% by weight or more.

**3.** The production method according to claim 1 or 2,
wherein the plant-derived carbon source contains 6 mM or less of butanoic acid.

**4.** The production method according to any one of claims 1 to 3,
wherein the plant-derived carbon source contains 35 mM or less of hexanoic acid.

**5.** The production method according to any one of claims 1 to 4,
wherein the plant-derived carbon source is treated with activated clay.

**6.** The production method according to any one of claims 1 to 5,
wherein the plant-derived carbon source is palm kernel oil.

**7.** The production method according to any one of claims 1 to 6,
wherein the microorganism is a bacterium of the genus Cupriavidus.

**8.** The production method according to claim 7, wherein the bacterium of the genus Cupriavidus is Cupriavidus necator or its recombinant.

**9.** The production method according to any one of claims 1 to 8, wherein the polyhydroxyalkanoate is polyhydroxybutyrate-hexanoate.

**10.** A method for producing a carbon source for culturing a microorganism, the method comprising:

contacting a plant-derived carbon source with activated clay.

## INTERNATIONAL SEARCH REPORT

International application No.
PCT/JP2013/073982

A. CLASSIFICATION OF SUBJECT MATTER
*C12P7/62*(2006.01)i, *C12N1/00*(2006.01)i

According to International Patent Classification (IPC) or to both national classification and IPC

B. FIELDS SEARCHED

Minimum documentation searched (classification system followed by classification symbols)
C12P7/62, C12N1/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JST7580(JDreamIII), CAplus/MEDLINE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2010/113497 A1 (Kaneka Corp.), 07 October 2010 (07.10.2010), claims; paragraph [0005]; example 2 & EP 2418277 A1 | 1-9<br>10 |
| X<br>Y | AZIZ, N. A., et al., Improvement of the production of poly(3-hydroxybutyrate-co-3-hydroxyvalerate-co-4-hydroxybutyrate) terpolyester by manipulating the culture condition., J. Chem. Technol. Biotechnol., Published online 30 April 2012, Vol.87, No.11, p.1607-1614 | 1-9<br>10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>07 October, 2013 (07.10.13) | Date of mailing of the international search report<br>15 October, 2013 (15.10.13) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2013/073982

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2005-6510 A (Mizusawa Industrial Chemicals, Ltd.), 13 January 2005 (13.01.2005), paragraph [0003] (Family: none) | 10 |
| Y | JP 2003-119486 A (Kaneka Corp.), 23 April 2003 (23.04.2003), paragraph [0002] (Family: none) | 10 |
| A | JP 2009-225775 A (Kaneka Corp.), 08 October 2009 (08.10.2009), entire text (Family: none) | 1-10 |
| A | WO 2009/145164 A1 (Kaneka Corp.), 03 December 2009 (03.12.2009), entire text & US 2011/0091948 A1 & EP 2295536 A1 | 1-10 |
| A | WO 2012/102371 A1 (Kaneka Corp.), 02 August 2012 (02.08.2012), entire text (Family: none) | 1-10 |
| A | SUDESH, K., et al., Synthesis of polyhydroxyalkanoate from palm oil and some new applications., Appl. Microbiol. Biotechnol., 2011, Vol.89, p.1373-1386 | 1-10 |
| A | LOO, C.-Y., et al., Biosynthesis and characterization of poly(3-hydroxybutyrate-co-3- hydroxyhexanoate) from palm oil products in a Wautersia eutropha mutant., Biotechnol. Lett., 2005, Vol.27, p.1405-1410 | 1-10 |

Form PCT/ISA/210 (continuation of second sheet) (July 2009)

**INTERNATIONAL SEARCH REPORT**

International application No.
PCT/JP2013/073982

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Document 1 (WO 2010/113497 A1) discloses that a polyhydroxyalkanoate is produced by culturing microorganisms using a long chain fatty acid such as lauric acid or myristic acid as a carbon source, and that the long chain fatty acid is derived from a plant. Consequently, the invention of claim 1 of this international application is not novel over document 1, and thus does not have a special technical feature.

Therefore, there is no same or corresponding special technical feature between the invention of claim 1 and the invention of claim 10.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007049716 A
- JP 2007259708 A
- WO 2009145164 A


### Non-patent literature cited in the description


- *Appl Microbiol Biotechnol,* 2011, vol. 89, 1373-1386
- *Biological Macromolecules,* 1999, vol. 25, 43-53
- *Journal of Oleo Science,* 2007, vol. 56 (1), 13-17
- **KATO et al.** *Appl. MicroBiol. Biotechnol.,* 1996, vol. 45, 363
- *Bull. Chem. Soc.,* 1996, vol. 69, 515
- Molecular Cloning. Cold Spring Harbor Laboratory Press, 1989
- **STERNBERG, J. C. ; GALLAWAY, W. E. ; JONES, D. T. L.** Gas Chromatography. Academic Press, 1962, 207
- **SIMON, R. ; PRIEFER, U. ; PUHLER, A.** A broad host range mobilization system for in vivo genetic engineering transposon mutagenesis in gram negative bacteria. *Biotechnology,* 1983, vol. 1, 784-791
- **WATANABE, Y. ; ICHINOMIYA, Y. ; SHIMADA, D. ; SAIKA. A. ; ABE, H. ; TAGUCHI, S. ; TSUGE, T.** Development and validation of an HPLC-based screening method to acquire polyhydroxyalkanoate synthase mutants with altered substrate specificity. *J Biosci Bioeng.,* 2011, vol. 113, 286-292